# EUROPÄISCHE PATENTSCHRIFT

(11) **EP 2 244 762 B1**
(45) Veröffentlichungstag und Bekanntmachung des Hinweises auf die Patenterteilung: **27.09.2017**
(21) Anmeldenummer: 09716013.9
(22) Anmeldetag: 27.02.2009
(51) Int. Cl.: A61M 1/28

(54) **VERFAHREN ZUR ÜBERPRÜFUNG UND/ODER ÜBERWACHUNG DER DICHTIGKEIT MEHRERER PNEUMATISCH ODER HYDRAULISCH BETÄTIGTER AKTOREN UND MASCHINE, INSBESONDERE MEDIZINISCHE BEHANDLUNGSMASCHINE**
METHOD FOR CHECKING AND/OR MONITORING THE TIGHTNESS OF A PLURALITY OF PNEUMATICALLY OR HYDRAULICALLY ACTUATED ACTUATORS, AND MACHINE, ESPECIALLY MEDICAL TREATMENT MACHINE
PROCÉDÉ DE CONTRÔLE ET/OU DE SURVEILLANCE DE L'ÉTANCHÉITÉ DE PLUSIEURS ACTIONNEURS À COMMANDE PNEUMATIQUE OU HYDRAULIQUE ET INSTRUMENT, NOTAMMENT INSTRUMENT DE TRAITEMENT MÉDICAL

(30) Priorität: 29.02.2008 DE 102008011822
(43) Veröffentlichungstag der Anmeldung: 03.11.2010
(73) Patentinhaber: Fresenius Medical Care Deutschland GmbH, 61352 Bad Homburg (DE)
(72) Erfinder: HEDMANN, Frank L., 97332 Volkach (DE); KLATTE, Stephan, 31582 Nienburg (Weser) (DE)
(74) Vertreter: Laufhütte, Dieter
(86) Internationale Anmeldenummer: PCT/EP2009/001434
(87) Internationale Veröffentlichungsnummer: WO 2009/106353

(56) Entgegenhaltungen:
- EP-A1- 0 856 321
- EP-A2- 0 293 592

## Beschreibung

Die vorliegende Erfindung betrifft ein Verfahren zur Überprüfung und/oder Überwachung der Dichtigkeit mehrerer pneumatisch oder hydraulisch betätigter Aktoren einer Maschine, insbesondere mehrerer Ventilaktoren einer medizinischen Behandlungsmaschine, sowie eine Maschine, insbesondere eine medizinische Behandlungsmaschine, mit einer Steuerung zur Durchführung der entsprechenden Verfahren.

Insbesondere betrifft die vorliegende Erfindung dabei ein Verfahren zur Überprüfung und/oder Überwachung der Dichtigkeit mehrerer pneumatisch oder hydraulisch betätigter Aktoren einer Maschine, bei welcher ein Kassettensystem zum Transport von insbesondere medizinischen Flüssigkeiten eingesetzt wird. Besonders vorteilhaft kommt das erfindungsgemäße Verfahren dabei im Bereich der Dialyse, insbesondere der Peritonealdialyse, zum Einsatz, insbesondere bei Behandlungsmaschinen mit einem Kassettensystem zum Transport der Behandlungsflüssigkeiten bzw. zur Durchführung der Behandlung. Ebenso kann das Verfahren in der Hämodialyse oder bei Infusionssystemen eingesetzt werden.

Insbesondere betrifft die vorliegende Erfindung dabei Peritonealdialysemaschinen, wie sie in US 2007/0112297 A1 und US 2006/0195064 A1 dargestellt sind, sowie Verfahren zum Betrieb solcher Peritonealdialysemaschinen.

Bei den pneumatisch oder hydraulisch betätigbaren Aktoren der Maschine handelt es sich dabei vorteilhafterweise um Ventilaktoren, mit welchen die in diesen Kassettensystemen verwendeten Ventile geschaltet werden. Die Kassetten sind dabei als Einwegartikel ausgeführt und weisen Ventilstellen auf, auf welche die Ventilaktoren der Maschine einwirken und so die Ventile schalten. Insbesondere weisen die Kassetten dabei flüssigkeitsführende Kanäle auf, welche im Bereich der Ventilstellen mindestens eine flexible Wand aufweisen, die durch den Ventilaktor in den flüssigkeitsführenden Kanal gedrückt werden kann und diesen so versperrt. Die Aktoren weisen dabei vorteilhafterweise einen flexiblen Bereich auf, welcher sich durch Beaufschlagen des Aktors mit Druck ausdehnt und so als Ventilstößel dient. Ebenso sind hydraulisch oder pneumatisch betätigbare Kolben als Aktoren einsetzbar, welche vorteilhafterweise ebenfalls als Ventilstößel dienen. Bei solchen Kassettensystemen ist damit der vorteilhafterweise kassetenseitige Bereich zum Fördern der Nutzflüssigkeit wie z. B. des Dialysats von dem vorteilhafterweise maschinenseitigen Bereich, welcher zur Betätigung der Aktoren mit Druck beaufschlagt wird, durch mindestens eine Membran getrennt.

Während des laufenden Betriebs der Maschine werden unterschiedliche Schaltmuster der Ventile durchlaufen, um die z.B. während eines Spülvorgangs oder einer Behandlung in der Kassette benötigten Flüssigkeitswege bereitzustellen. Die Aktoren der Maschine werden hierzu während des laufenden Betriebs der Maschine in unterschiedlichen Kombinationen mit Druck beaufschlagt, um so die entsprechenden Ventile in der Kassette entsprechend zu schalten und die gewünschten Fluidwege für den Betrieb der Kassette bereitzustellen.

Die Aktoren haben dabei üblicherweise einen aktiven Schaltzustand, bei welchem sie mit Druck beaufschlagt sind, und einen inaktiven Schaltzustand, bei welchem sie nicht mit Druck beaufschlagt sind. Zwischen diesen Zuständen kann durch beaufschlagen der Aktoren mit Druck bzw. Ablassen des Drucks hin und her geschaltet werden. Während solcher Umschaltprozesse ändert sich dabei der am System anliegende Druck, während sich zwischen den Umschaltprozessen ein stationärer Zustand einstellt, bei welchem der an den mit Druck beaufschlagten Aktoren anliegende Druck im wesentlichen konstant ist. Bei den pneumatisch oder hydraulisch betätigbaren Aktoren kann es allerdings auf der mit Druck beaufschlagten Seite zu Leckagen kommen.

Bei bekannten Verfahren zur Überprüfung und/oder Überwachung der Dichtigkeit solcher Aktoren wird daher üblicherweise ein Initialtest zu Beginn der Behandlung durchgeführt, bei welchem die Aktoren mit Druck beaufschlagt werden und im sich anschließenden stationären Zustand der Druckabfall pro Zeiteinheit gemessen wird, um Undichtigkeiten festzustellen. Übersteigt der Druckabfall pro Zeiteinheit dabei einen gewissen Grenzwert, wird der Betrieb des Gerätes nicht freigegeben. Ein solches Verfahren ist aus der Druckschrift EP 856 321 A1 bekannt, bei welchem zunächst alle Ventilaktuatoren mit negativem Druck beaufschlagt werden und ein Dichtigkeitswert bestimmt wird. Übersteigt dieser einen Grenzwert, so wird ein Systemfehler erkannt und das Gerät abgeschaltet. Der gleiche Vorgang wird mit positivem Druck wiederholt. Daraufhin werden entsprechende Tests mit den Pumpaktuatoren durchgeführt.

Mit einem solchen Verfahren lässt sich zwar eine bei der Initialisierung des Gerätes vorliegende Undichtigkeit erkennen. Während des Betriebes des Gerätes ergibt sich jedoch keine Möglichkeit mehr, festzustellen, ob eine Leckage im System entstanden ist. Insbesondere kann während des Betriebs der Maschine kein Initialtest durchgeführt werden, da hierzu die Maschine komplett gestoppt werden müßte. Des weiteren gibt es keinen Anhaltspunkt, wann ein geeigneter Zeitpunkt für einen solchen Test während der Behandlung wäre. Undichtigkeiten der Aktoren können aber gerade während der laufenden Behandlung durch einen Verschleiß der Aktoren entstehen.

Aufgabe der vorliegenden Erfindung ist es daher, ein Verfahren zur Überprüfung und/oder Überwachung der Dichtigkeit mehrerer pneumatisch oder hydraulisch betätigter Aktoren zur Verfügung zu stellen, welches während des laufenden Betriebs der Maschine durchgeführt werden kann. Weiterhin ist es Aufgabe der vorliegenden Erfindung, eine Maschine, insbesondere eine medizinische Behandlungsmaschine, mit einer entsprechenden Ventilaktorüberwachung zur Verfügung zu stellen. Erfindungsgemäß wird diese Aufgabe von einem Verfahren gemäß Anspruch 1, einer Maschine gemäß Anspruch 12 und einem Computerprogrammprodukt gemäß Anspruch 21 gelöst. Vorteilhafte Ausgestaltungen der Erfindung sind Gegenstand der Unteransprüche.

Die Erfindung umfaßt dabei ein Verfahren zur Überprüfung und/oder Überwachung der Dichtigkeit mehrerer pneumatisch oder hydraulisch betätigter Aktoren einer Maschine, insbesondere mehrerer Ventilaktoren einer medizinischen Behandlungsmaschine, wobei die Aktoren während des normalen Betriebs der Maschine in unterschiedlichen Kombinationen mit Druck beaufschlagt werden. Erfindungsgemäß wird dabei für mehrere unterschiedliche Kombinationen von mit Druck beaufschlagten Aktoren der während einer stationären Betriebsphase an den in der jeweiligen Kombination mit Druck beaufschlagten Aktoren gemeinsam auftretende Druckabfall gemessen. Für einzelne Aktoren und/oder einzelne Gruppen von Aktoren wird dann jeweils ein Dichtigkeitswert ermittelt, in welchen der für jene Kombinationen gemessene Druckabfall eingeht, bei welchen der jeweilige Aktor und/oder die jeweilige Gruppe von Aktoren mit Druck beaufschlagt wurde.

Die erfindungsgemäße Maschine beaufschlagt dabei während ihres Betriebs die Aktoren in unterschiedlichen Kombinationen mit Druck, um auf der Kassettenseite die Ventile zu schalten und so die gewünschten Flüssigkeitswege herzustellen. Hierdurch ergeben sich Schalt-Betriebsphasen, in welchen zwischen unterschiedlichen Kombinationen von mit Druck beaufschlagten Aktoren umgeschaltet wird, so dass der Systemdruck schwankt, und dazwischen liegende stationäre Betriebsphasen, in welchen an einer festen Kombination von Aktoren ein im wesentlichen konstanter Systemdruck anliegt, welcher nicht durch Umschaltvorgänge verändert wird und lediglich durch die Undichtigkeit des Systems mit der Zeit abnehmen kann. Der Druckabfall in diesen stationären Betriebsphasen ist daher ein Maß für die Dichtigkeit der gerade mit Druck beaufschlagten Aktoren.

Während dieser stationären Betriebsphasen wird nun jeweils der an allen mit Druck beaufschlagten Aktoren gemeinsamen anliegende Druck gemessen und der während dieser stationären Betriebsphasen entstehende Druckabfall bestimmt. Der Druckabfall wird dabei vorteilhafterweise für den Zeitraum gemessen, in dem eine entsprechende Kombination besteht. Insbesondere kann zu zwei unterschiedlichen Zeitpunkten während der stationären Betriebsphase, insbesondere zu Beginn und am Ende der stationären Betriebsphase, der an allen mit Druck beaufschlagten Aktoren gemeinsamen anliegende Druck gemessen und aus der Differenz der beiden Werte der Druckabfall bestimmt werden. Bei einem Umschaltvorgang wird der gemessene Druckabfall dann vorteilhafterweise einer Auswerteeinheit zugeführt und vorteilhafterweise in einer Tabelle abgelegt.

Eine Einzelmessung des Druckabfalls für nur eine Kombination von mit Druck beaufschlagten Aktoren lässt dabei üblicherweise keine sicheren Rückschlüsse auf die Dichtigkeit einzelner Aktoren und/oder einzelner Gruppen von Aktoren zu, da bei einer solchen Kombination üblicherweise mehrere Aktoren gleichzeitig mit Druck beaufschlagt sind und der gemessene Druckabfall somit einen Sammelwert für die Summe aller Undichtigkeiten der beteiligten Aktoren bildet.

Aus den Daten für mehrere unterschiedliche Kombinationen von mit Druck beaufschlagten Aktoren kann nun aber auch für einzelne Aktoren und/oder einzelne Gruppen von Aktoren ein Dichtigkeitswert ermittelt werden. Dabei geht der für eine bestimmte Kombination gemessene Druckabfall in den Dichtigkeitswert eines spezifischen Aktors ein, wenn dieser Aktor in dieser Kombination mit Druck beaufschlagt war. War ein Aktor dagegen in einer bestimmten Kombination nicht mit Druck beaufschlagt, so geht der während dieser Kombination gemessene Druckabfall auch nicht in den Dichtigkeitswert dieses Aktors ein.

Aus den sich hierdurch ansammelnden Werten kann damit zumindest nach einer gewissen Zeit bzw. Häufigkeit der Messungen eine Tendenz für einen Aktor bzw. für eine Gruppe von Aktoren festgestellt werden. Obwohl also während des üblichen laufenden Betriebs der Maschine üblicherweise mehrere Aktoren gleichzeitig mit Druck beaufschlagt werden, kann so durch die Ansammlung von Werten aus mehreren unterschiedlichen Kombinationen die Dichtigkeit jedes einzelnen Aktors beurteilt werden. Erfindungsgemäß wird hierbei ausgenutzt, dass bei dem Betrieb der Maschine ohnehin eine Vielzahl von unterschiedlichen Kombinationen von Aktoren mit Druck beaufschlagt wird. Durch Auswertung des Druckabfalls für diese unterschiedlichen Kombinationen lässt sich so eine Einschätzung der Dichtigkeit der einzelnen Aktoren gewinnen.

Vorteilhafterweise geht dabei in den Dichtigkeitswert neben dem Druckabfall jeweils die Messzeit ein, während welcher der Druckabfall aufgetreten ist. Bei dem erfindungsgemäßen Verfahren werden demnach neben dem für die jeweilige Kombination auftretenden Druckabfall auch die Messzeit, während welcher dieser Druckabfall aufgetreten ist, berücksichtigt. Hierfür werden vorteilhafterweise der Druckabfall und die Messzeit für die jeweilige stationäre Betriebsphase einer Auswerteinheit übergeben. Da der während einer stationären Betriebsphase auftretende Druckabfall neben der Undichtigkeit der jeweils mit Druck beaufschlagten Aktoren auch von der Messzeit abhängt, ist durch die Berücksichtigung dieser Messzeiten eine genauere Detektion von Undichtigkeiten möglich.

Insbesondere kann dabei in den Dichtigkeitswert ein aus den gemessenen Druckabfallswerten und den Messzeiten gebildeter Druckabfall pro Zeiteinheit eingehen. Der Druckabfall pro Zeiteinheit bildet dabei ein relativ genaues Mass für die Dichtigkeit der jeweiligen Aktoren.

Vorteilhafterweise wird dabei zur Bestimmung des Dichtigkeitswerts eines einzelnen Aktors und/oder einer einzelnen Gruppe von Aktoren ein Mittelwert gebildet, in welchen der Druckabfall und vorteilhafterweise die Messzeit für all jene Kombinationen von mit Druck beaufschlagten Aktoren eingeht, bei welchen der jeweilige Aktor und/oder die jeweilige Gruppe von Aktoren mit Druck beaufschlagt wurde. Es wird also zur Bestimmung des Dichtigkeitswerts eines bestimmten Aktors ein Mittelwert aus all jenen Meßwerten für den Druckabfall gebildet, welche in Betriebsphasen gemessen wurden, in denen dieser bestimmte Aktor mit Druck beaufschlagt wurde. Obwohl jeder einzelne Aktor in diesen Betriebsphasen gegebenenfalls nur einen gewissen Anteil am Druckabfall selbst hervorgerufen hat, während ein anderer Teil des Druckabfalls über weitere, ebenfalls mit Druck beaufschlagte Aktoren hervorgerufen wurde, ergibt sich durch diese Mittelung über mehrere unterschiedliche Kombinationen von Aktoren für jeden einzelnen Aktor ein relativ gut angenäherter Wert für die Dichtigkeit. Vorteilhafterweise gehen dabei jeweils auch die Messzeiten für den jeweiligen Druckabfall in die Mittelwertbildung ein, wobei insbesondere ein Mittelwert des gemessenen Druckabfalls pro Zeiteinheit gebildet werden kann.

Vorteilhafterweise wird dabei zur Bestimmung des Dichtigkeitswertes eines einzelnen Aktors und/oder einer einzelnen Gruppe von Aktoren der gegebenenfalls gewichtete Mittelwert des Druckabfalls und/oder des Druckabfalls pro Zeiteinheit all jener Kombinationen von mit Druck beaufschlagten Aktoren berechnet, bei welchen der jeweilige Aktor oder die jeweilige Gruppe von Aktoren mit Druck beaufschlagt wurde.

Die gegebenenfalls gewichtete Mittelwertbildung kann dabei auf unterschiedliche Art und Weise erfolgen. Insbesondere können dabei alle Kombinationen, bei welchen ein Aktor mit Druck beaufschlagt wurde, gleich gewichtet werden, so dass der für eine gewisse Kombination gemessene Druckabfall und/oder Druckabfall pro Zeiteinheit unabhängig von der Dauer und der Anzahl der mit Druck beaufschlagten Aktoren in die Mittelwertbildung für den Dichtigkeitswert der beteiligten Aktoren eingeht.

Vorteilhafterweise geht jedoch die Dauer der stationären Betriebsphasen, in welchen der Druckabfall und/oder Druckabfall pro Zeiteinheit gemessen wurde, in die Mittelwertbildung ein. Insbesondere kann dabei für den Dichtigkeitswert eines spezifischen Aktors der zeitliche Mittelwert des während all der stationären Betriebsphasen gemessenen Druckabfalls pro Zeiteinheit gebildet werden, in welchen dieser Aktor mit Druck beaufschlagt wurde. Hierdurch werden Kombinationen, welche über eine längere Dauer hinweg bestehen, stärker gewichtet, als Kombinationen, welche nur kurzzeitig geschaltet sind. Bei Kombinationen mit längeren stationären Betriebsphasen sind jedoch auch die Meßwerte für den Druckabfall pro Zeiteinheit genauer, so dass sich hierdurch insgesamt die Genauigkeit erhöht.

Vorteilhafterweise wird dabei zur Bestimmung des Dichtigkeitswerts eines einzelnen Aktors und/oder einer einzelnen Gruppe von Aktoren die Summe der Druckabfälle und die Summe der Messzeiten für all jene Kombinationen von mit Druck beaufschlagten Aktoren bestimmt, bei welchen der jeweilige Aktor und/oder die jeweilige Gruppe von Aktoren mit Druck beaufschlagt wurde. Aus dem Quotienten dieser beiden Summen kann dann ein mittlerer Druckabfall pro Zeiteinheit bestimmt werden, welcher in den Dichtigkeitswert des Aktors und/oder der Gruppe von Aktoren eingeht.

Weiterhin kann vorteilhafterweise die Anzahl der in einer bestimmten Kombination mit Druck beaufschlagten Aktoren in die Mittelwertbildung eingehen. Insbesondere kann dabei der für eine bestimmte Kombination ermittelte Druckabfall um so geringer in die Dichtigkeitswerte der beteiligten Aktoren eingehen, je mehr Aktoren mit Druck beaufschlagt wurden. Hierdurch wird verhindert, dass Kombinationen mit sehr vielen mit Druck beaufschlagten Aktoren, bei welchen entsprechend hohe gemeinsame Druckverluste auftreten, überproportional stark in die Mittelwertbildung einfließen.

Alternativ kann jedoch auch vorgesehen sein, dass der für eine bestimmte Kombination von mit Druck beaufschlagten Aktoren gemessene gemeinsame Druckabfall unabhängig von der Anzahl der in dieser Kombination mit Druck beaufschlagten Aktoren in die Mittelwertbildung eingeht. Hierdurch wird der einem Aktor mit hoher Leckrate zugeordnete Dichtigkeitswert nicht dadurch vermindert, dass dieser Aktor zusammen in Kombination mit anderen Aktoren betrieben wird, welche eine erheblich niedrigere Leckrate aufweisen. Durch eine solche Kombination sind demnach defekte Aktoren besonders gut zu erkennen, verschlechtern jedoch auch die Werte der eigentlich korrekt arbeitenden Aktoren.

Weiterhin vorteilhafterweise geht erfindungsgemäß der für eine bestimmte Kombination von mit Druck beaufschlagten Aktoren gemessene gemeinsame Druckabfall für alle in der jeweiligen Kombination mit Druck beaufschlagten Aktoren in gleicher Höhe in deren Dichtigkeitswert ein. Eine solche gleichmäßige Verteilung der Werte ist insbesondere dann sinnvoll, wenn alle Aktoren identisch aufgebaut sind.

Weiterhin vorteilhafterweise werden erfindungsgemäß die Dichtigkeitswerte der einzelnen Aktoren und/oder einzelnen Gruppen von Aktoren während des Betriebs der Maschine laufend aktualisiert. Insbesondere werden die Dichtigkeitswerte der einzelnen Aktoren und/oder einzelnen Gruppen von Aktoren vorteilhafterweise dann aktualisiert, wenn die Maschine von einer Kombination von mit Druck beaufschlagten Aktoren in eine andere Kombination umschaltet. Der während der stationären Betriebsphase der vorherigen Kombination gemessene Druckabfall bzw. Druckabfall pro Zeiteinheit wird dann vorteilhafterweise zur Aktualisierung der Dichtigkeitswerte der einzelnen Aktoren und/oder einzelnen Gruppen von Aktoren herangezogen, während bei Erreichen der stationären Betriebsphase der neuen Kombination eine neue Druckmessung begonnen wird.

Zur Überprüfung und/oder Überwachung der Aktoren wird bei dem erfindungsgemäßen Verfahren vorteilhafterweise die Bandbreite der bestimmten Dichtigkeitswerte herangezogen. Es werden dabei der niedrigste Dichtigkeitswert mit dem höchsten Dichtigkeitswert verglichen, und so die Bandbreite der Dichtigkeitswerte über alle Aktoren bestimmt. Eine niedrige Bandbreite ist dabei ein Indikator für eine hohe Dichtigkeit der Aktoren, während eine hohe Bandbreite ein Indikator für eine Leckage ist, da offensichtlich zumindest ein Aktor vorliegt, der einen erheblich anderen Dichtigkeitswert aufweist als die übrigen Aktoren.

Weiterhin vorteilhafterweise wird zur Überprüfung und/oder Überwachung der Aktoren die Summe aller bestimmten Dichtigkeitswerte herangezogen. Auch diese Summe erlaubt eine globale Aussage über die Dichtigkeit des Systems.

Weiterhin vorteilhafterweise wird zur Überprüfung und/oder Überwachung der Aktoren die Veränderung über die Zeit der bestimmten Dichtigkeitswerte herangezogen. Hierdurch lässt sich ermitteln, ob bei einzelnen Aktoren oder einzelnen Gruppen von Aktoren starke Veränderungen der Dichtigkeit auftreten.

Durch Auswerten der entsprechenden Dichtigkeitswerte kann somit eine Leckage während des laufenden Betriebs der Maschine erkannt werden. Vorteilhafterweise wird dabei bei einem Erkennen einer Leckage der laufende Betrieb gestoppt und ein Test durchgeführt. Durch einen solchen Test kann nun noch einmal sichergestellt werden, dass es sich bei den während des laufenden Betriebs ermittelten Werten nicht um eine Fehlmessung handelt, sondern dass tatsächlich eine Leckage vorliegt.

Dabei kann als Test ein Initialtest durchgeführt werden, bei welchem alle Aktoren überprüft werden. Eine solche Initialtest-Routine ist bei bekannten Geräten bereits vorgesehen und wird initial vor Inbetriebnahme durchgeführt.

Alternativ kann auch ein Test durchgeführt werden, bei welchem nur der einzelne Aktor und/oder die einzelne Gruppe von Aktoren, bei welcher bzw. bei welchen eine Leckage erkannt wurde, überprüft wird. Hierdurch ist es möglich, die während des laufenden Betrieb festgestellte Leckage schnell zu überprüfen. Bestätigt sich die Leckage bei dem einzelnen Aktor bzw. bei der einzelnen Gruppe von Aktoren nicht, kann gegebenenfalls noch ein Initialtest durchgeführt werden, um zu überprüfen, ob nicht bei anderen Aktoren eine Leckage vorliegt.

Weiterhin vorteilhafterweise wird bei Erkennen und/oder Bestätigung einer Leckage der undichte Aktor nicht weiter verwendet oder die Maschine in einen sicheren Zustand gebracht. Da viele Fluidwege in einer Kassette durch Schalten unterschiedlicher Aktoren bereitgestellt werden können, ist es möglich, während des laufenden Betriebs einzelne Aktoren nicht zu betätigen, und dennoch die Maschine weiterzubetreiben. Erfindungsgemäß können daher als undicht erkannte Aktoren ohne eine Beeinträchtigung der Sicherheit der Kassette still gelegt werden. Ist dies nicht möglich, schaltet die Maschine dagegen in einen sicheren Zustand, bis eine Reparatur erfolgt ist.

Allgemein können dann, wenn eine Leckage erkannt wurde, Sicherheitsmaßnahmen eingeleitet werden. Insbesondere kann dabei auch ein Alarm ausgelöst werden.

Vorteilhafterweise wird die erfindungsgemäße Maschine zum Ansteuern der Ventile einer Kassette zum Transport einer medizinischen Flüssigkeit eingesetzt, insbesondere in der Dialyse. Solche Kassetten weisen dabei üblicherweise flüssigkeitsführende Kanäle auf, welche im Bereich der Ventilstellen mindestens eine flexible Wand aufweisen, welche zum Absperren der Kanäle in diese hineingedrückt wird. Insbesondere sind die flüssigkeitsführenden Kanäle dabei über eine flexible Membran abgedeckt. Durch Drücken dieser Membran in die flüssigkeitsführenden Kanäle können diese dann abgesperrt werden, so dass sich Ventile ergeben. Die erfindungsgemäßen Aktoren arbeiten dabei vorteilhafterweise als Ventilstößel, um die flexible Wand bzw. Membran in die flüssigkeitsführenden Kanäle hineinzudrücken. Insbesondere kann ein erfindungsgemäßer Aktor dabei einen flexiblen Bereich aufweisen, welcher sich bei Anlegen von Druck ausdehnt und so in die flüssigkeitsführenden Bereiche der Kassette hineingedrückt wird.

Die vorliegende Erfindung umfasst weiterhin eine Maschine, insbesondere eine medizinische Behandlungsmaschine, mit mehreren pneumatisch oder hydraulisch betätigbaren Aktoren, insbesondere mehreren Ventilaktoren, und mit einem Druckmeßgerät zur Messung des an den mit Druck beaufschlagten Aktoren gemeinsam anliegenden Drucks, sowie mit einer elektronischen Steuerung zur insbesondere automatischen Durchführung eines Verfahrens, wie es oben beschrieben wurde. Die erfindungsgemäße elektronische Steuerung weist dabei vorteilhafterweise eine entsprechend ausgeführte Recheneinheit sowie einen Speicher zum Ablegen der Dichtigkeitswerte auf. Durch eine solche Maschine ergeben sich offensichtlich die gleichen Vorteile, welche weiter oben im Bezug auf das Verfahren dargestellt wurden. Insbesondere kann die Maschine das erfindungsgemäße Verfahren zur Überprüfung bzw. Überwachung der Dichtigkeit der Aktoren der Maschine automatisch während des laufenden Betriebs der Maschine ausführen, so dass sich die Sicherheit des Betriebs erhöht.

Vorteilhafterweise handelt es sich bei der Maschine dabei um eine Maschine zum Ansteuern der Ventile einer Kassette zum Transport einer medizinischen Flüssigkeit, und zwar insbesondere um eine Maschine zum Einsatz in der Dialyse, insbesondere in der Peritonealdialyse. In eine solche Maschine wird dabei die Kassette als Einwegelement eingelegt und dient dem Transport einer medizinischen Flüssigkeit, z. B. des Dialysats. Die erfindungsgemäßen Aktoren werden dann an die Kassette angekoppelt und als Ventilaktoren zum Ansteuern der Ventile der Kassette eingesetzt.

Die vorliegende Erfindung umfasst damit eine Maschine, insbesondere eine medizinische Behandlungsmaschine, mit mehreren pneumatisch oder hydraulisch betätigbaren Aktoren, insbesondere mehreren Ventilaktoren, mit einem Druckmessgerät zur Messung des an den mit Druck beaufschlagten Aktoren gemeinsam anliegenden Drucks und mit einer elektronischen Steuerung, welche die Aktoren und das Druckmessgerät so ansteuert, dass die Aktoren während des normalen Betriebs der Maschine in unterschiedlichen Kombinationen mit Druck beaufschlagt werden und für mehrere unterschiedliche Kombinationen von mit Druck beaufschlagten Aktoren der während einer stationären Betriebsphase an den in der jeweiligen Kombination mit Druck beaufschlagten Aktoren gemeinsam auftretende Druckabfall gemessen wird. Weiterhin umfasst die Maschine dabei eine Auswerteeinheit, welche für einzelne Aktoren und/oder einzelne Gruppen von Aktoren jeweils einen Dichtigkeitswert ermittelt, in welchen der für jene Kombinationen gemessene Druckabfall eingeht, in welchen der jeweilige Aktor und/oder die jeweilige Gruppe von Aktoren mit Druck beaufschlagt wurde. Weiterhin ist eine Überprüfungs- und/oder Überwachungseinheit vorgesehen, welche die Dichtigkeit der Aktoren der Maschine auf Grundlage der ermittelten Dichtigkeitswerte überprüft und/oder überwacht. Auch die erfindungsgemäße Maschine hat damit die Möglichkeit, während des laufenden Betriebs die Dichtigkeit der pneumatisch oder hydraulisch betätigten Aktoren laufend zu überwachen, ohne dass hierfür der Betrieb der Maschine unterbrochen werden müßte.

Vorteilhafterweise geht dabei in die von der Auswerteeinheit gebildeten Dichtigkeitswerte neben dem Druckabfall jeweils die Meßzeit ein, während welcher der Druckabfall aufgetreten ist. Da der Druckabfall, welcher während einer stationären Betriebsphase an den in der jeweiligen Kombination mit Druck beaufschlagten Aktoren gemeinsam auftritt, neben den Undichtigkeiten der jeweils mit Druck beaufschlagten Aktoren auch von der Meßzeit, während welcher der Druckabfall bestimmt wird, abhängt, ergibt sich damit eine genauere Bestimmung der Dichtigkeitswerte. Insbesondere kann dabei der Druck während zweier unterschiedlicher Zeitpunkte einer stationären Betriebsphase gemessen werden und der hierdurch bestimmte Druckabfall zusammen mit der Zeitspanne zwischen den beiden Zeitpunkten der Auswerteeinheit übergeben werden. Ebenfalls kann ein Speicher vorgesehen sein, in welchem diese Werte oder daraus berechnete Werte abgespeichert werden.

Vorteilhafterweise geht dabei in den Dichtigkeitswert ein aus den gemessenen Druckabfallswerten und dem Meßzeiten gebildeter Druckabfall pro Zeiteinheit ein. Der Druckabfall pro Zeiteinheit ist dabei ein relativ genaues Maß für die Dichtigkeit eines Aktors und/oder einer einzelnen Gruppe von Aktoren. Dabei kann sowohl der jeweils für die unterschiedlichen Kombinationen von mit Druck beaufschlagten Aktoren gemessene Druckabfall pro Zeiteinheit in die Auswertung eingehen, und/oder aus den gemessenen Druckabfallswerten und Meßzeiten für eine Vielzahl von Kombinationen ein mittlerer Druckabfall pro Zeiteinheit bestimmt werden.

Vorteilhafterweise bildet dabei die Auswerteeinheit zur Bestimmung des Dichtigkeitswerts eines einzelnen Aktors und/oder einer einzelnen Gruppe von Aktoren einen Mittelwert, in welchen der Druckabfall und vorteilhafterweise die Meßzeit für all jene Kombinationen von mit Druck beaufschlagten Aktoren eingeht, bei welchen der jeweilige Aktor und/oder die jeweilige Gruppe von Aktoren mit Druck beaufschlagt wurde. Durch die Mittelwertbildung über die Meßwerte für all die Kombinationen, in welchen ein bestimmter Aktor oder eine bestimmte Gruppe von Aktoren mit Druck beaufschlagt wurde, ergibt sich somit eine gute Annäherung an den tatsächlichen Dichtigkeitswert des jeweiligen Aktores oder die jeweilige Gruppe von Aktoren.

Vorteilhafterweise berechnet dabei die Auswerteeinheit den Dichtigkeitswert eines einzelnen Aktors und/oder einer einzelnen Gruppe von Aktoren als den gegebenenfalls gewichteten Mittelwert des Druckabfalls und/oder des Druckabfalls pro Zeiteinheit all jener Kombinationen von mit Druck beaufschlagten Aktoren, bei welchen der jeweilige Aktor oder die jeweilige Gruppe von Aktoren mit Druck beaufschlagt wurde. Die Gewichtung der Meßwerte für den Druckabfall und/oder den Druckabfall pro Zeiteinheit für die jeweiligen Kombination von mit Druck beaufschlagten Aktoren kann dabei auf unterschiedliche Art und Weise erfolgen.

Insbesondere kann dabei die Dauer der stationären Betriebsphasen, für welche der Druckabfall und/oder der Druckabfall pro Zeiteinheit bestimmt wurde, in die Mittelwertbildung eingehen. Insbesondere kann dann, wenn ein Mittelwert der gemessenen Druckabfälle pro Zeiteinheit gebildet wird, wenn also die Meßzeiten ohnehin in die Dichtigkeitswerte eingehen, zusätzlich hierzu die Dauer der stationären Betriebsphasen bzw. die Meßzeiten als Faktoren in die Mittelwertbildung eingehen. Insbesondere kann dabei der zeitliche Mittelwert gebildet werden.

Weiterhin kann die Anzahl der in einer bestimmten Kombination mit Druck beaufschlagten Aktoren in die Mittelwertbildung eingehen. Alternativ hierzu kann der für eine bestimmte Kombination von mit Druck beaufschlagten Aktoren gemessene gemeinsame Druckabfall unabhängig von der Anzahl der in dieser Kombination mit Druck beaufschlagten Aktoren in die Mittelwertbildung eingehen. Hierdurch sind undichte Aktoren leichter zu erkennen, beeinflussen jedoch auch die Dichtigkeitswerte eigentlich dichter Aktoren stärker.

Vorteilhafterweise geht weiterhin der für eine bestimmte Kombination von mit Druck beaufschlagten Aktoren gemessene gemeinsame Druckabfall für alle in der jeweiligen Kombination mit Druck beaufschlagten Aktoren in gleicher Höhe in deren Dichtigkeitswert ein. Dieses Vorgehen ist darin begründet, dass das System keine näheren Informationen dazu hat, wie ein während einer stationären Phase auftretender gemeinsamer Druckabfall mehrerer mit Druck beaufschlagter Aktoren auf die einzelnen Aktoren zurückgeht. Durch die Verwendung von Meßwerten mehrerer unterschiedlicher Kombinationen von Aktoren ergibt sich dennoch für jeden einzelnen Aktor oder für jede bestimmte Gruppe von Aktoren mit der Zeit eine relativ gute Annäherung an die tatsächliche Dichtigkeit des einzelnen Aktors oder der Gruppe von Aktoren.

Zur konkreten Umsetzung des erfindungsgemäßen Verfahrens kann dabei vorteilhafterweise die Auswerteeinheit zur Bestimmung des Dichtigkeitswerts eines einzelnen Aktors und/oder einer einzelnen Gruppe von Aktoren die Summe der Druckabfälle und die Summe der Meßzeiten für all jene Kombinationen von mit Druck beaufschlagten Aktoren bestimmen, bei welchen der jeweilige Aktor und/oder die jeweilige Gruppe von Aktoren mit Druck beaufschlagt wurde. Aus dieser Summe der Druckabfälle und dieser Summe der Meßzeiten kann dann für jeden Aktor oder für jede Gruppe von Aktoren ein mittlerer Druckabfall pro Zeiteinheit bestimmt werden, aus welchem sich wiederum der Dichtigkeitswert des jeweiligen Aktors oder der jeweiligen Gruppe von Aktoren ergibt. Ein solches Vorgehen entspricht der Bildung des zeitlichen Mittelwertes über den Druckabfall pro Zeiteinheit all jener Kombinationen von mit Druck beaufschlagten Aktoren, bei welchen der jeweilige Aktor und/oder die jeweilige Gruppe von Aktoren mit Druck beaufschlagt wurde.

Weiterhin vorteilhafterweise aktualisiert erfindungsgemäß die Auswerteeinheit die Dichtigkeitswerte der einzelnen Aktoren und/oder einzelnen Gruppen von Aktoren während des Betriebes der Maschine laufend. Hierdurch ergibt sich eine sichere Überprüfung und/oder Überwachung der Dichtigkeit der Aktoren.

Vorteilhafterweise überprüft und/oder überwacht die Überprüfungs- und/oder Überwachungseinheit die Dichtigkeit der Aktoren dabei auf Grundlage der Bandbreite der bestimmten Dichtigkeitswerte. Eine hohe Bandbreite der Dichtigkeitswerte ist dabei ein Indikator für einen undichten Aktor, während eine niedrige Bandbreite ein Indikator für die Dichtigkeit aller Aktoren ist.

Weiterhin vorteilhafterweise überprüft und/oder überwacht die Überprüfungs- und/oder Überwachungseinheit die Dichtigkeit der Aktoren auf Grundlage der Summe aller bestimmten Dichtigkeitswerte. Diese Summe aller bestimmten Dichtigkeitswerte ist dabei ein Wert, welcher der Gesamtdichtigkeit des Systems entspricht.

Weiterhin vorteilhafterweise überprüft und/oder überwacht die Überprüfungs- und/oder Überwachungseinheit der erfindungsgemäßen Maschine die Dichtigkeit der Aktoren auf Grundlage der Veränderung über die Zeit der bestimmten Dichtigkeitswerte. Eine solche Veränderung der bestimmten Dichtigkeitswerte kann dabei ein Indikator für das Auftreten einer Leckage sein.

Offensichtlich kann dabei zur Überprüfung und/oder Überwachung der Dichtigkeit der Aktoren auch eine Kombination mehrerer Werte herangezogen werden.

Weiterhin vorteilhafterweise stoppt die Überprüfungs- und/oder Überwachungseinheit beim Erkennen einer Leckage den laufenden Betrieb der Maschine und führt einen Test durch. Durch diesen Test kann dann z. B. die durch die Überprüfungs- und/oder Überwachungseinheit während des laufenden Betriebs festgestellte Undichtigkeit eines gewissen Aktors oder einer gewissen Gruppe von Aktoren noch einmal überprüft bzw. bestätigt werden. Vorteilhafterweise kann dabei erfindungsgemäß ein Initialtest durchgeführt werden, bei welchem alle Aktoren überprüft werden. Alternativ kann jedoch ein Test durchgeführt werden, bei welchem ein einzelner Aktor und/oder eine einzelne Gruppe von Aktoren, bei welcher eine Leckage erkannt wurde, überprüft wird. Es müssen damit nicht mehr alle Aktoren überprüft werden, da zur Bestätigung der Leckage zunächst eine Überprüfung des Aktors oder der Gruppe von Aktoren ausreicht, bei welcher die Überprüfungs- und/oder Überwachungseinheit die Leckage erkannt hatte.

Vorteilhafterweise verwendet die Steuerung der erfindungsgemäßen Maschine bei einem Erkennen und/oder einer Bestätigung einer Leckage den undichten Aktor nicht weiter. Insbesondere kann die Steuerung dabei den Betrieb der Maschine fortsetzen, wenn dies unter Umgehung des undichten Aktors möglich ist. Andernfalls schaltet die Maschine in einen sicheren Zustand

Vorteilhafterweise wird die erfindungsgemäße Maschine dabei zum Ansteuern der Ventile einer Kassette zum Transport einer medizinischen Flüssigkeit, insbesondere zum Einsatz in der Dialyse eingesetzt. Die Kassette bildet dabei üblicherweise ein Einwegteil, welches in die Maschine eingesetzt wird, wobei die Maschine die Flüssigkeitsströme in der Kassette ansteuert.

Vorteilhafterweise weist die erfindungsgemäße Maschine dabei eine Ankoppelfläche, an welche eine Kassette zum Transport einer medizinischen Flüssigkeit ankoppelbar ist, auf, wobei die Aktoren an der Ankoppelfläche angeordnet sind. So können die maschinenseitigen Aktoren auf die Ventile der angekoppelten Kassette eingreifen und so unterschiedliche Fluidwege in der Kasse zur Verfügung stellen. Die vorliegende Erfindung umfasst weiterhin vorteilhafterweise ein Computerprogrammprodukt, insbesondere ein Speichermedium mit einem Computerprogramm, insbesondere zum Aufspielen auf eine Maschine, mit Befehlen zur Durchführung eines Verfahrens, wie es oben beschrieben wurde. Auch durch ein solches Computerprogrammprodukt ergeben sich die gleichen Vorteile, wie sie bezüglich des Verfahrens beschrieben wurden. Insbesondere kann ein solches auf einem Speichermedium gespeichertes Computerprogramm auf eine bestehende Maschine aufgespielt werden, um dort das erfindungsgemäße Verfahren zur Überprüfung und/oder Überwachung der Dichtigkeit der Aktoren der Maschine durchzuführen. Hierdurch können bestehende Maschinen mit dem erfindungsgemäßen Verfahren nachgerüstet werden. Dies ist insbesondere dadurch problemlos möglich, dass zur Durchführung des Verfahrens keine zusätzlichen Komponenten benötigt werden.

Die vorliegende Erfindung wird nun anhand eines Ausführungsbeispiels sowie Zeichnungen näher beschrieben. Dabei zeigen:

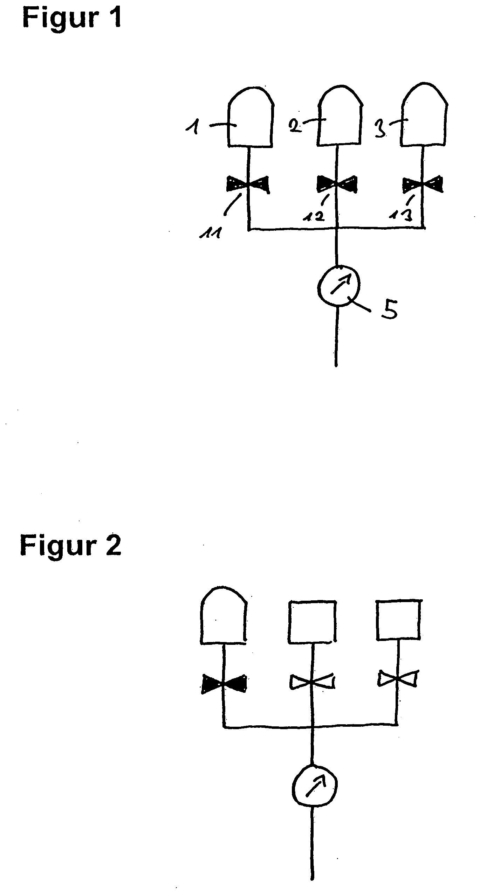

- Figuren 1 - 4:: unterschiedliche Kombinationen von mit Druck beaufschlagten Aktoren während des Betriebs einer erfindungsgemäßen Maschine.

Die vorliegende Erfindung wird nun anhand eines Ausführungsbeispiels näher dargestellt, bei welchem das erfindungsgemäße Verfahren zur Überprüfung bzw. Überwachung der Dichtigkeit mehrerer pneumatisch betätigter Ventilaktoren einer medizinischen Behandlungsmaschine zum Einsatz kommt.

In solchen medizinischen Behandlungsmaschinen werden üblicherweise Einmalkassetten zum Transport von medizinischen Flüssigkeiten verwendet. Diese Einmalkassetten weisen Flüssigkeitskanäle auf, welche von einer flexiblen Folie abgedeckt sind. Die Einwegkassette wird dabei in die Behandlungsmaschine eingelegt und so an eine geräteseitige Ankoppelfläche angekoppelt, dass die in der Ankoppelfläche der Behandlungsmaschine angeordneten Aktoren die flexible Folie der Kassette in die flüssigkeitsführenden Kanäle hineindrücken können und so die Fluidwege in der Kassette bestimmen können. Die pneumatisch betätigten Aktoren weisen dabei vorteilhafterweise flexible Bereiche auf, welche sich bei Anlegen von Druck an den Aktor ausdehnen und so die flexible Folie in die flüssigkeitsführenden Kanäle drücken. Hierdurch können neben Ventilen auch Pumpen und ähnliches realisiert werden.

Während des laufenden Betriebs der Behandlungsmaschine werden dabei unterschiedliche Kombinationen von Aktoren abwechselnd mit Druck beaufschlagt, um entsprechend unterschiedliche Fluidwege in der Kassette bereitzustellen. Dabei sind typischerweise in jeder Kombination mehrere Ventile gleichzeitig mit Druck beaufschlagt, so dass der während einer spezifischen Kombination an allen mit Druck beaufschlagten Ventilen gemeinsam auftretende Druckverlust nicht einem einzelnen Ventil zugeordnet werden kann. Zur Überprüfung der Dichtigkeit der einzelnen Aktoren der erfindungsgemäßen Behandlungsmaschine wird nun das erfindungsgemäße Verfahren eingesetzt, welches während des normalen Betriebs der Behandlungsmaschine durchgeführt wird. Durch das erfindungsgemäße Verfahren ist es dabei möglich, durch die Verwendung von Daten aus mehreren unterschiedlichen Kombinationen von mit Druck beaufschlagten Aktoren jedem einzelnen Aktor einen entsprechenden Dichtigkeitswert zuzuordnen.

Dabei wird zunächst für mehrere unterschiedliche Kombinationen von mit Druck beaufschlagten Aktoren der während einer stationären Betriebsphase an den in der jeweiligen Kombination mit Druck beaufschlagten Aktoren gemeinsam auftretende Druckabfall gemessen und zusammen mit der Messzeit gespeichert. Aus diesen Daten wird nun für einzelne Aktoren und/oder einzelne Gruppen von Aktoren jeweils ein Dichtigkeitswert ermittelt, in welchen der für jene Kombinationen gemessene Druckabfall und/oder Druckabfall pro Zeiteinheit eingeht, in denen der jeweilige Aktor und/oder die jeweilige Gruppe von Aktoren mit Druck beaufschlagt wurde. Der Verlauf des Betriebsdruckes im System lässt sich dabei in zwei Phasen unterteilen: In einer ersten Phase ergibt sich ein veränderlicher Druckbereich, da von einer Kombination von mit Druck beaufschlagten Ventilen zu einer anderen Kombination geschaltet wird, so dass entsprechend Druck aufgebaut werden muss. Zwischen diesen Umschalt-Phasen ergeben sich stationäre Betriebsphasen, in welchen keine Aktionen ausgeführt werden. In diesen stationären Betriebsphasen liegt ein stabiler Druck vor, welcher nun erfindungsgemäß gemessen wird. Dabei wird für die unterschiedlichen Kombinationen jeweils der Druckabfall während der mit einer spezifischen Kombination verbundenen stationären Betriebsphase gemessen und zusammen mit der Messzeit einer Auswerteeinheit übergeben. Der während einer solchen stationären Betriebsphase gemessene Druckabfall und die Messzeit wird dann all jenen Aktoren zugeordnet, welche während dieser spezifischen Kombination mit Druck beaufschlagt waren. Der jedem einzelnen Aktor zugeordnete Dichtigkeitswert ergibt sich so im Laufe des Betriebs aus dem gemittelten Druckabfall der Kombinationen, an welchen der jeweilige Aktor beteiligt war.

In den Figuren 1 bis 4 sind nun zur Veranschaulichung unterschiedliche Kombinationen von mit Druck beaufschlagten Aktoren gezeigt. Dabei werden stellvertretend für die üblicherweise erheblich höhere Anzahl von Aktoren (z.B. 16 Aktoren) in einer Behandlungsmaschine die Aktoren 1, 2 und 3 gezeigt, welche jeweils über Vorrichtungen 11, 12 und 13 mit Druck beaufschlagt bzw. von der Druckversorgung getrennt werden können. Die Vorrichtungen 11, 12 und 13 sind dabei schwarz ausgefüllt gezeigt, wenn die zugehörigen Aktoren 1, 2 oder 3 mit Druck beaufschlagt sind, bzw. weiß ausgemalt, wenn die entsprechenden Aktoren nicht mit Druck beaufschlagt sind. Dabei ist eine zentrale Druckversorgung für alle mit Druck beaufschlagten Aktoren vorgesehen, deren Druck über den Druckmesser 5 bestimmt werden kann.

In Figur 1 ist dabei eine erste Kombination gezeigt, bei welcher alle Aktoren mit Druck beaufschlagt sind. In Figur 2 ist dagegen lediglich Aktor 1 mit Druck beaufschlagt, während die Aktoren 2 und 3 inaktiv sind. In Figur 3 sind die Aktoren 1 und 2 mit Druck beaufschlagt, während der Aktor 3 inaktiv ist. In Figur 4 ist Aktor 1 inaktiv, während die Aktoren 2 und 3 mit Druck beaufschlagt sind.

In der Tabelle ist nun tabellarisch gezeigt, wie durch die Messung des Druckabfalls und die Zuordnung der Werte zu den jeweils mit Druck beaufschlagten Aktoren die Leckrate der einzelnen Aktoren während des laufenden Betriebs der Behandlungsmaschine bestimmt werden kann. Dabei ergibt sich durch Beaufschlagung unterschiedlicher Kombinationen von Aktoren jeweils ein Ventilmuster in der Kassette.

Aus der jeweils verwendeten Kombination von mit Druck beaufschlagten Aktoren ergibt sich dann für jede Kombination eine gewisse gemeinsame Leckrate der aktiven Aktoren. Das Ausführungsbeispiel geht dabei von einer Leckrate der den Ventilen V1 bis V3 zugeordneten Aktoren 1 bis 3 von
V1 : 5 mbar/min
V2 : 15 mbar/min
V3 : 0 mbar/min
aus. Dabei wird nach einem Umschalten der Aktoren zunächst abgewartet, bis sich ein stationärer Betriebszustand eingestellt hat. Daraufhin wird im Ausführungsbeispiel der Druck so lange gemessen, wie die entsprechende Kombination besteht. Bei einem Wechsel des Ventilmusters mit einer entsprechend neuen Kombination von mit Druck beaufschlagten Aktoren wird der für die vorangegangene Kombination gemessene Druckabfall zusammen mit der Messzeit einer Auswerteeinheit zugeführt und z. B. in einer Tabelle abgelegt, wobei die Messwerte nur den in der entsprechenden Kombination mit Druck beaufschlagten Aktoren zugeordnet werden. Daraufhin wird für alle Aktoren der Mittelwert der ihnen zugeordneten Messwerte gebildet.

Der jedem Aktor zugeordnete Dichtigkeitswert entspricht damit dem zeitlichen Mittelwert des gemeinsamen Druckabfalls pro Zeiteinheit aller bisherigen Kombinationen, in welchen der jeweilige Aktor mit Druck beaufschlagt war.

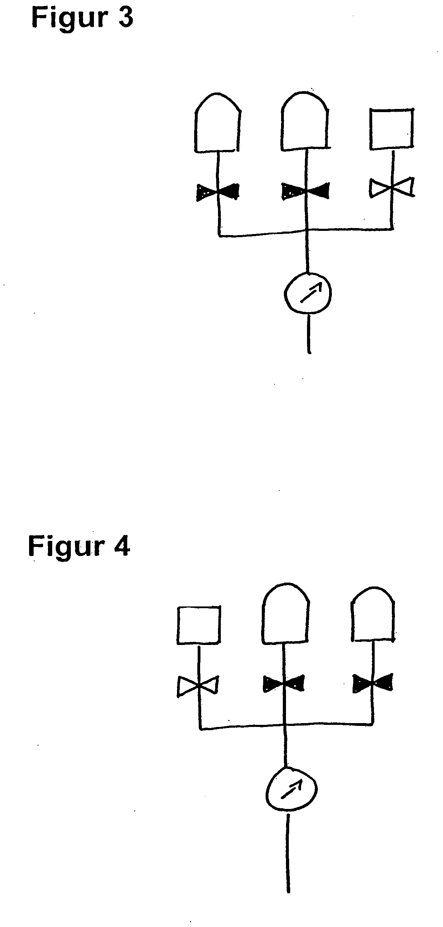

Bei dem in der Tabelle gezeigten Muster 1, welches einer Anordnung gemäß Figur 1 entspricht, sind dabei alle Aktoren mit Druck beaufschlagt und dem entsprechend alle Ventile V1 bis V3 geschlossen. Hierdurch ergibt sich eine gemeinsame Leckrate aller Aktoren von 20 mbar/min. Der Druckabfall während des Bestehens des Muster 1 wird dabei gemessen und zusammen mit der aktuellen Meßzeit für alle beteiligten Aktoren in der Tabelle abgelegt. Daraufhin wird zu Muster 2 geschaltet, welches Figur 2 entspricht, bei welchem lediglich Aktor 1 mit Druck beaufschlagt ist. Hierdurch ergibt sich eine Leckrate von 5 mbar/min. Der entsprechende Druckabfall und die Meßzeit werden dementsprechend auch bloß Aktor 1 zugeordnet. Bei Muster 3, welches Figur 3 entspricht, sind dann die ersten beiden Aktoren mit Druck beaufschlagt, wodurch sich eine gemeinsame Leckrate von 20 mbar/min ergibt. Der hierdurch entstehende Druckabfall zusammen mit den Meßzeiten wird dann für die beteiligten Aktoren 1 und 2 in der Tabelle abgelegt.

Wird nun jeweils der gesamte einem Aktor zugeordnete Druckabfall durch die gesamte einem Aktor zugeordnete Meßzeit geteilt, ergibt sich ein mittlerer Druckabfall pro Zeiteinheit für jeden Aktor. Je mehr Kombinationen dabei in einen solchen Dichtigkeitswert einfließen, desto stärker wird die tatsächliche Leckrate angenähert. Dabei sind jedoch undichte Ventile deutlicher zu erkennen als dichte Ventile, da die dichten Ventile in ihrem Ergebnis von den undichten Ventilen beeinflußt werden.

Dies lässt sich auch in der Auswertung z. B. nach Muster 7 erkennen, bei welcher Aktor 1 eine Leckrate von 10 mbar/min, Aktor 2 eine Leckrate von 16,7 mbar/min und Aktor 3 eine Leckrate von 5 mbar/min zugeordnet wird.

Das in der Tabelle gezeigte Verfahren zur Bestimmung der Dichtigkeitswerte der einzelnen Aktoren lässt sich dabei besonders einfach umsetzen, da hier der jeweils gemessene Druckabfall über die Dauer der stationären Betriebsphasen allen während dieser Betriebsphasen mit Druck beaufschlagten Aktoren in gleicher Weise zugeordnet wird, ohne dass die Anzahl von mit Druck beaufschlagten Ventilen hierbei berücksichtigt würde. Als Mittelwert ergibt sich dann für jeden Aktor das zeitliche Mittel über all die Betriebsphasen, in welchen der Aktor mit Druck beaufschlagt war. Hierdurch wird sichergestellt, dass die für jeden einzelnen Aktor als Dichtigkeitswert ermittelte mittlere Leckrate immer mindestens genauso hoch ist wie die tatsächliche Leckrate dieses Aktors.

Alternativ sind jedoch auch andere Mittelwertbildungen denkbar, bei welchen z. B. keine Mittelung über die Zeit erfolgt, sondern der Druckabfall pro Zeiteinheit, welcher für die unterschiedlichen Kombinationen von Aktoren bestimmt wird, in jeweils gleicher Weise in das Endergebnis eingeht. Ebenso ist es denkbar, die Anzahl der mit Druck beaufschlagten Aktoren in die Mittelung einfließen zu lassen.

Durch das erfindungsgemäße Verfahren ist es möglich, auch für eine Vielzahl von Aktoren eine zuverlässige Erkennung von Leckagen während des laufenden Betriebs der Behandlungsmaschine zu gewährleisten. Eine typische Anzahl von Aktoren beträgt dabei z. B. 16. Erfindungsgemäß können so alle 16 Ventile während des laufenden Betriebs über einen einzigen Druckmesser überwacht werden, indem die während unterschiedlicher Kombinationen von geschalteten Aktoren ermittelten Druckabfallwerte jeweils den aktiven Aktoren zugeordnet werden.

Hierbei ergibt sich ein Ergebnisvektor, in welchem der Dichtigkeitswert für jeden einzelnen Aktor abgespeichert und jeweils beim Wechseln von einer Kombination in eine andere Kombination aktualisiert wird. Durch die häufige Wiederholung des erfindungsgemäßen Meßverfahrens mit unterschiedlichen Mustern ergibt sich so eine Annäherung an die tatsächliche Gesamtsituation des Systems.

Der Ergebnisvektor kann dann zur Leckageerkennung weiterverarbeitet werden. So kann z. B. mit Hilfe einer Minimum-/Maximumbewertung des Ergebnisvektors die Bandbreite der Leckrate bestimmt werden. Eine (schmale Bandbreite, bei welcher alle Aktoren ähnliche Dichtigkeitsraten aufweisen, ist dabei ein Indikator für ein ordnungsgemäß funktionierendes System. Eine weite Bandbreite deutet dagegen auf eine mögliche Leckage hin. Weiterhin kann mit Hilfe einer Summenbewertung des Ergebnisvektors und dessen Gradienten über die Zeit eine Änderung im Leckageverhalten erkannt werden. Ebenso kann mit Hilfe einer Minimalwertbildung eine Grundleckrate des Systems ermittelt werden.

Durch die Auswertung ist es möglich, einen defekten Aktor zu erkennen, da dieser Aktor für jede Kombination, bei welcher er mit Druck beaufschlagt ist, eine erhöhte Leckagerate verursacht. Diese schlägt sich dann im erhöhten Mittelwert für diesen Aktor nieder. Hierbei ist zu beachten, dass erst über die Häufigkeit der Einzelmessungen pro Komponente ein Trend zu erkennen ist. Meßungenauigkeiten, welche bei schnellen Ventilwegwechseln und damit schnellen Wechseln von Aktuatorkombinationen auftreten können, werden dabei herausgefiltert.

Entdeckt das System für einen Aktor einen Dichtigkeitswert, welcher als Indikator für eine Leckage gewertet wird, kann zusätzlich ein Test zur Überprüfung des Ergebnisses durchgeführt werden. Hierzu wird der Betrieb des Behandlungsgerätes unterbrochen und eine Testroutine durchgeführt. Dabei kann es sich entweder um einen bekannten Initialtest handeln, bei welchem alle Aktoren überprüft werden. Alternativ kann auch zunächst nur der Aktor oder die Gruppe von Aktoren überprüft werden, bei welcher die Leckage aufgrund des entsprechenden Dichtigkeitswertes, welcher außerhalb eines zulässigen Bereichs lag, erkennt wurde.

Ist ein Aktor als undicht identifiziert worden, kann die Behandlungsmaschine in einen sicheren Zustand wechseln. Sind jedoch ausreichend Alternativen für den defekten Aktor vorhanden, so kann der Betrieb auch unter Umgehung dieses Aktors weitergeführt werden.

Durch das erfindungsgemäße Verfahren ist es damit möglich, während des laufenden Betriebs der Behandlungsmaschine ständig alle Aktoren auf ihre Dichtigkeit zu überwachen und gegebenenfalls zu reagieren.

**Tabelle**

| | offen geschlossen + | Aktuelfer Druckabfalt | Aktuelle Messzeit | Summe Druckabfall | Summe Messzeit | Auswertung Mbar/min: |
|---|---|---|---|---|---|---|
| Muster 1 | V1 + | 20 mbar | 1 min | 20 mbar | 1 min | 20 |
| | V2 + | 20 mbar | 1 min | 20 mbar | 1 min | 20 |
| | V3 + | 20 mbar | 1 min | 20 mbar | 1 min | 20 |
| Muster 2 | V1 + | 25 mbar | 5 min | 45 mbar | 6 min | 7,5 |
| | V2 - | ---- | ---- | 20 mbar | 1 min | 20 |
| | V3 - | ---- | ---- | 20 mbar | 1 min | 20 |
| Muster 3 | V1 + | 40 mbar | 2 min | 85 mbar | 8 min | 10,6 |
| | V2 + | 40 mbar | 2 min | 60 mbar | 3 min | 20 |
| | V3 - | ---- | ---- | 20 mbar | 1 min | 20 |
| Muster 4 | V1 - | ---- | ---- | 85 mbar | 8 min | 10,6 |
| | V2 + | 15 mbar | 1 min | 75 mbar | 4 min | 18,8 |
| | V3 + | 15 mbar | 1 min | 35 mbar | 2 min | 17,5 |
| Muster 5 | V1 - | ---- | ---- | 85 mbar | 8 min | 10,6 |
| | V2 - | ---- | ---- | 75 mbar | 4 min | 18,8 |
| | V3 + | 0 mbar | 5 min | 35 mbar | 7 min | 5 |
| Muster 6 | V1 + | 5 mbar | 1 min | 90 mbar | 9 min | 10 |
| | V2 - | ---- | ---- | 75 mbar | 4 min | 18,8 |
| | V3 + | 5 mbar | 1 min | 40 mbar | 8 min | 5 |
| Muster 7 | V1 - | ---- | ---- | 90 mbar | 9 min | 10 |
| | V2 + | 75 mbar | 5 min | 150 mbar | 9 min | 16,7 |
| | V3 - | ---- | ---- | 40 mbar | 8 min | 5 |

## Patentansprüche

1. Verfahren zur Überprüfung und/oder Überwachung der Dichtigkeit mehrerer pneumatisch oder hydraulisch betätigter Aktoren einer Maschine, insbesondere mehrerer Ventilaktoren einer medizinischen Behandlungsmaschine,
wobei die Aktoren während des normalen Betriebs der Maschine in unterschiedlichen Kombinationen mit Druck beaufschlagt werden,
**dadurch gekennzeichnet,**
**dass** für mehrere unterschiedliche Kombinationen von mit Druck beaufschlagten Aktoren der während einer stationären Betriebsphase an den in der jeweiligen Kombination mit Druck beaufschlagten Aktoren gemeinsam auftretende Druckabfall gemessen wird und
wobei für einzelne Aktoren und/oder einzelne Gruppen von Aktoren jeweils ein Dichtigkeitswert ermittelt wird, in welchen der für jene Kombinationen gemessene Druckabfall eingeht, in welchen der jeweilige Aktor und/oder die jeweilige Gruppe von Aktoren mit Druck beaufschlagt wurde.

2. Verfahren nach Anspruch 1, wobei in den Dichtigkeitswert neben dem Druckabfall jeweils die Meßzeit eingeht, während welcher der Druckabfall aufgetreten ist und wobei vorzugsweise in den Dichtigkeitswert ein aus den gemessenen Druckabfallswerten und den Meßzeiten gebildeter Druckabfall pro Zeiteinheit eingeht.

3. Verfahren nach einem der vorangegangenen Ansprüche, wobei zur Bestimmung des Dichtigkeitswerts eines einzelnen Aktors und/oder einer einzelnen Gruppe von Aktoren ein Mittelwert gebildet wird, in welchen der Druckabfall und vorteilhafterweise die Meßzeit für all jene Kombinationen von mit Druck beaufschlagten Aktoren eingeht, bei welchen der jeweilige Aktor und/oder die jeweilige Gruppe von Aktoren mit Druck beaufschlagt wurde.

4. Verfahren nach Anspruch 3, wobei zur Bestimmung des Dichtigkeitswertes eines einzelnen Aktors und/oder einer einzelnen Gruppe von Aktoren der gegebenenfalls gewichtete Mittelwert des Druckabfalls und/oder des Druckabfalls pro Zeiteinheit all jener Kombinationen von mit Druck beaufschlagten Aktoren berechnet wird, bei welchen der jeweilige Aktor oder die jeweilige Gruppe von Aktoren mit Druck beaufschlagt wurde, wobei vorzugsweise die Dauer der stationären Betriebsphasen, für welche der Druckabfall und/oder der Druckabfall pro Zeiteinheit bestimmt wurde, in die Mittelwertbildung eingeht.

5. Verfahren nach einem der vorangegangenen Ansprüche, wobei zur Bestimmung des Dichtigkeitswerts eines einzelnen Aktors und/oder einer einzelnen Gruppe von Aktoren die Summe der Druckabfälle und die Summe der Meßzeiten für all jene Kombinationen von mit Druck beaufschlagten Aktoren bestimmt wird, bei welchen der jeweilige Aktor und/oder die jeweilige Gruppe von Aktoren mit Druck beaufschlagt wurde.

6. Verfahren nach Anspruch 4, wobei die Anzahl der in einer bestimmten Kombination mit Druck beaufschlagten Aktoren in die Mittelwertbildung eingeht oder wobei der für eine bestimmte Kombination von mit Druck beaufschlagten Aktoren gemessene gemeinsame Druckabfall unabhängig von der Anzahl der in dieser Kombination mit Druck beaufschlagten Aktoren in die Mittelwertbildung eingeht.

7. Verfahren nach einem der vorangegangenen Ansprüche, wobei der für eine bestimmte Kombination von mit Druck beaufschlagten Aktoren gemessene gemeinsame Druckabfall für alle in der jeweiligen Kombination mit Druck beaufschlagten Aktoren in gleicher Höhe in deren Dichtigkeitswert eingeht und wobei vorteilhaft die Dichtigkeitswerte der einzelnen Aktoren und/oder einzelnen Gruppen von Aktoren während des Betriebs der Maschine laufend aktualisiert werden, wobei zur Überprüfung und/oder Überwachung der Aktoren weiter vorteilhaft die Bandbreite der bestimmten Dichtigkeitswerte herangezogen wird oder wobei zur Überprüfung und/oder Überwachung der Aktoren andererseits vorteilhaft die Summe aller bestimmten Dichtigkeitswerte herangezogen wird.

8. Verfahren nach einem der vorangegangenen Ansprüche, wobei zur Überprüfung und/oder Überwachung der Aktoren die Veränderung über die Zeit der bestimmten Dichtigkeitswerte herangezogen wird.

9. Verfahren nach einem der vorangegangenen Ansprüche, wobei bei einem Erkennen einer Leckage der laufende Betrieb gestoppt und ein Test durchgeführt wird, wobei vorzugsweise ein Initialtest durchgeführt wird, bei welchem alle Aktoren überprüft werden, und/oder wobei weiter vorteilhaft ein Test durchgeführt wird, bei welchem ein einzelner Aktor und/oder eine einzelne Gruppe von Aktoren, bei welcher eine Leckage erkannt wurde, überprüft wird.

10. Verfahren nach einem der vorangegangenen Ansprüche, wobei bei einem Erkennen und/oder einer Bestätigung einer Leckage der undichte Aktor nicht weiter verwendet wird oder die Maschine in einen sicheren Zustand wechselt.

11. Verfahren nach einem der vorangegangenen Ansprüche, wobei die Maschine zum Ansteuern der Ventile einer Kassette zum Transport einer medizinischen Flüssigkeit eingesetzt wird, insbesondere in der Dialyse.

12. Maschine, insbesondere medizinische Behandlungsmaschine, mit mehreren pneumatisch oder hydraulisch betätigbaren Aktoren, insbesondere mehreren Ventilaktoren, mit einem Druckmeßgerät zur Messung des an den mit Druck beaufschlagten Aktoren gemeinsam anliegenden Drucks und mit einer elektronischen Steuerung, welche die Aktoren und das Druckmeßgerät so ansteuert,
dass die Aktoren während des normalen Betriebs der Maschine in unterschiedlichen Kombinationen mit Druck beaufschlagt werden und für mehrere unterschiedliche Kombinationen von mit Druck beaufschlagten Aktoren der während einer stationären Betriebsphase an den in der jeweiligen Kombination mit Druck beaufschlagten Aktoren gemeinsam auftretende Druckabfall gemessen wird,
wobei eine Auswerteeinheit vorgesehen ist, welche für einzelne Aktoren und/oder einzelne Gruppen von Aktoren jeweils ein Dichtigkeitswert ermittelt, in welchen der für jene Kombinationen gemessene Druckabfall eingeht, in welchen der jeweilige Aktor und/oder die jeweilige Gruppe von Aktoren mit Druck beaufschlagt wurde, und
wobei eine Überprüfungs- und/oder Überwachungseinheit die Dichtigkeit der Aktoren der Maschine auf Grundlage der ermittelten Dichtigkeitswerte überprüft und/oder überwacht.

13. Maschine nach einem der vorangegangenen Ansprüche, wobei die Auswerteeinheit zur Bestimmung des Dichtigkeitswerts eines einzelnen Aktors und/oder einer einzelnen Gruppe von Aktoren ein Mittelwert bildet, in welchen der Druckabfall und vorteilhafterweise die Meßzeit für all jene Kombinationen von mit Druck beaufschlagten Aktoren eingeht, bei welchen der jeweilige Aktor und/oder die jeweilige Gruppe von Aktoren mit Druck beaufschlagt wurde, wobei die Auswerteeinheit vorzugsweise den Dichtigkeitswert eines einzelnen Aktors und/oder einer einzelnen Gruppe von Aktoren als den gegebenenfalls gewichteten Mittelwert des Druckabfalls und/oder des Druckabfalls pro Zeiteinheit all jener Kombinationen von mit Druck beaufschlagten Aktoren berechnet, bei welchen der jeweilige Aktor oder die jeweilige Gruppe von Aktoren mit Druck beaufschlagt wurde, wobei wahlweise vorzugsweise die Dauer der stationären Betriebsphasen, für welche der Druckabfall und/oder der Druckabfall pro Zeiteinheit bestimmt wurde, in die Mittelwertbildung eingeht und wobei wahlweise die Anzahl der in einer bestimmten Kombination mit Druck beaufschlagten Aktoren in die Mittelwertbildung eingeht oder wobei wahlweise der für eine bestimmte Kombination von mit Druck beaufschlagten Aktoren gemessene gemeinsame Druckabfall unabhängig von der Anzahl der in dieser Kombination mit Druck beaufschlagten Aktoren in die Mittelwertbildung eingeht.

14. Maschine nach einem der vorangegangenen Ansprüche, wobei der für eine bestimmte Kombination von mit Druck beaufschlagten Aktoren gemessene gemeinsame Druckabfall für alle in der jeweiligen Kombination mit Druck beaufschlagten Aktoren in gleicher Höhe in deren Dichtigkeitswert eingeht.

15. Maschine nach einem der vorangegangenen Ansprüche, wobei die Auswerteeinheit zur Bestimmung des Dichtigkeitswerts eines einzelnen Aktors und/oder einer einzelnen Gruppe von Aktoren die Summe der Druckabfälle und die Summe der Meßzeiten für all jene Kombinationen von mit Druck beaufschlagten Aktoren bestimmt, bei welchen der jeweilige Aktor und/oder die jeweilige Gruppe von Aktoren mit Druck beaufschlagt wurde.

16. Maschine nach einem der vorangegangenen Ansprüche, wobei die Überprüfungs- und/oder Überwachungseinheit bei einem Erkennen einer Leckage den laufende Betrieb stoppt und einen Test durchführt, wobei vorzugsweise ein Initialtest durchgeführt wird, bei welchem alle Aktoren überprüft werden.

17. Maschine nach Anspruch 16, wobei ein Test durchgeführt wird, bei welchem ein einzelner Aktor und/oder eine einzelne Gruppe von Aktoren, bei welcher eine Leckage erkannt wurde, überprüft wird.

18. Maschine nach einem der vorangegangenen Ansprüche, wobei die Steuerung bei einem Erkennen und/oder einer Bestätigung einer Leckage den undichten Aktor nicht weiter verwendet oder die Maschine in einen sicheren Zustand schaltet.

19. Maschine nach einem der vorangegangenen Ansprüche zum Ansteuern der Ventile einer Kassette zum Transport einer medizinischen Flüssigkeit, insbesondere zum Einsatz in der Dialyse.

20. Maschine nach einem der vorangegangenen Ansprüche mit einer Ankoppelfläche, an welche eine Kassette zum Transport einer medizinischen Flüssigkeit ankoppelbar ist, wobei die Aktoren an der Ankoppelfläche angeordnet sind.

21. Computerprogrammprodukt, insbesondere Speichermedium mit einem Computerprogramm, insbesondere zum Aufspielen auf eine Maschine, mit Befehlen zur Durchführung eines Verfahrens gemäß einem der vorangegangenen Ansprüche.

## Claims

1. A method for checking and/or monitoring the leak tightness of a plurality of pneumatically or hydraulically actuated actuators of a machine, in particular a plurality of valve actuators of a medical treatment machine,
wherein pressure is exerted on the actuators in different combinations during the operation of the machine;
**characterized in that**
the common pressure drop occurring during a stationary operating phase at the actuators on which pressure is exerted in the respective combination is measured for a plurality of different combinations of actuators on which pressure is exerted; and
wherein for individual actuators and/or individual groups of actuators, a leak tightness value is respectively determined into which the pressure drop is taken which is measured for those combinations in which pressure was exerted on the respective actuator and/or the respective group of actuators.

2. A method in accordance with claim 1, wherein the respective measuring time during which the pressure drop has occurred is taken into the leak tightness value in addition to the pressure drop and wherein a pressure drop per time unit formed from the measured pressure drop values and the measuring times is preferably taken into the leak tightness value.

3. A method in accordance with one of the preceding claims, wherein, for the determination of the leak tightness value of an individual actuator and/or of an individual group of actuators, a mean value is formed into which the pressure drop and advantageously the measuring time for all those combinations of actuators on which pressure is exerted are taken in which pressure was exerted on the respective actuator and/or the respective group of actuators.

4. A method in accordance with claim 3, wherein, for the determination of the leak tightness value of an individual actuator and/or of an individual group of actuators, the optionally weighted mean value of the pressure drop and/or of the pressure drop per time unit of all those combinations of actuators on which pressure is exerted is calculated in which pressure is exerted on the respective actuator or the respective group of actuators, wherein the duration of the stationary operating phases for which the pressure drop and/or the pressure drop per time unit was determined is preferably taken into the mean value formation.

5. A method in accordance with one of the preceding claims, wherein, for the determination of the leak tightness value of an individual actuator and/or of an individual group of actuators, the sum of the pressure drops and the sum of the measuring times for all those combinations of actuators on which pressure is exerted are advantageously determined at which pressure is exerted on the respective actuator and/or on the respective group of actuators.

6. A method in accordance with claim 4, wherein the number of the actuators on which pressure is exerted in a specific combination is taken into the mean value formation or wherein the measured common pressure drop for a specific combination of actuators on which pressure is exerted is taken into the mean value formation independently of the number of the actuators on which pressure is exerted in this combination.

7. A method in accordance with one of the preceding claims, wherein the common pressure drop measured for a specific combination of actuators on which pressure is exerted is taken into the leak tightness value for all actuators in a specific combination on which pressure is exerted in equal amounts and wherein the leak tightness values of the individual actuators and/or individual groups of actuators are advantageously continuously updated during the operation of the machine, wherein, further advantageously, the range of the determined leak tightness values is used for checking and/or monitoring the actuators or wherein the sum of all determined leak tightness values is advantageously used for checking and/or monitoring the actuators.

8. A method in accordance with one of the preceding claims, wherein the change of the determined leak tightness values over time is used for checking and/or monitoring the actuators.

9. A method in accordance with one of the preceding claims, wherein the ongoing operation is stopped and a test is carried out on a recognition of a leak, wherein an initial test is preferably carried out in which all the actuators are checked and/or wherein, further advantageously, a test is carried out in which an individual actuator and/or an individual group of actuators in which a leak was recognized is checked.

10. A method in accordance with one of the preceding claims, wherein, on the recognition and/or confirmation of a leak, the leaking actuator is no longer used or the machine changes into a safe state.

11. A method in accordance with one of the preceding claims, wherein the machine is used for the control of the valves of a cassette for the transport of a medical liquid, in particular in dialysis.

12. A machine, in particular a medical treatment machine, having a plurality of pneumatically or hydraulically actuable actuators, in particular a plurality of valve actuators, having a pressure gage for the measurement of the common pressure applied to the actuators on which pressure is exerted and having an electronic control which controls the actuators and the pressure gage such that pressure is exerted on the actuators in different combinations during the operation of the machine and the common pressure drop occurring during a stationary operating phase at the actuators on which pressure is exerted in the respective combination is measured for a plurality of different combinations of actuators on which pressure is exerted,
wherein an evaluation unit is provided which, for individual actuators and/or individual groups of actuators, determines a respective leak tightness value into which the pressure drop is taken which is measured for those combinations in which pressure was exerted on the respective actuator and/or the respective group of actuators; and
wherein a checking and/or monitoring unit checks and/or monitors the leak tightness of the actuators of the machine based on the determined leak tightness values.

13. A machine in accordance with one of the preceding claims, wherein the evaluation unit for the determination of the leak tightness value of an individual actuator and/or of an individual group of actuators, forms a mean value into which the pressure drop and advantageously the measuring time for all those combinations of actuators on which pressure is exerted are taken in which pressure was exerted on the respective actuator and/or the respective group of actuators, wherein the evaluation unit preferably calculates the leak tightness value of an individual actuator and/or of an individual group of actuators as the optionally weighted mean value of the pressure drop and/or of the pressure drop per time unit of all those combinations of actuators on which pressure is exerted in which pressure is exerted on the respective actuator or the respective group of actuators, wherein, optionally, the duration of the stationary operating phases for which the pressure drop and/or the pressure drop per time unit was determined is preferably taken into the mean value formation and wherein the number of the actuators on which pressure is exerted in a specific combination is optionally taken into the mean value formation or wherein the common pressure drop measured for a specific combination of actuators on which pressure is exerted is optionally taken into the mean value formation independently of the number of the actuators on which pressure is exerted in this combination.

14. A machine in accordance with one of the preceding claims, wherein the common pressure drop measured for a specific combination of actuators on which pressure is exerted is taken into the leak tightness value of said actuators in equal amounts for all actuators on which pressure is exerted in the respective combination.

15. A machine in accordance with one of the preceding claims, wherein the evaluation unit for the determination of the leak tightness value of an individual actuator and/or an individual group of actuators determines the sum of the pressure drops and the sum of the measuring times for all those combinations of actuators on which pressure is exerted in which pressure was exerted on the respective actuator and/or the respective group of actuators.

16. A machine in accordance with one of the preceding claims, wherein the checking and/or monitoring unit stops the ongoing operation on a recognition of a leak and carries out a test, wherein an initial test is preferably carried out in which all the actuators are checked.

17. A machine in accordance with claim 16, wherein a test is carried out in which an individual actuator and/or an individual group of actuators is checked in which a leak was recognized.

18. A machine in accordance with one of the preceding claims, wherein the control, on a recognition and/or a confirmation of a leak, no longer uses the leaking actuator or switches the machine into a safe state.

19. A machine in accordance with one of the preceding claims for the control of the valves of a cassette for the transport of a medical liquid, in particular for use in dialysis.

20. A machine in accordance with one of the preceding claims having a coupling surface to which a cassette can be coupled for the transport of a medical liquid, with the actuators being arranged at the coupling surface.

21. A computer program product, in particular a storage medium having a computer program, in particular for transfer to a machine, having commands for the carrying out of a method in accordance with one of the preceding claims.

## Revendications

1. Procédé de contrôle et/ou de surveillance de l'étanchéité de plusieurs actionneurs à commande pneumatique ou hydraulique d'un instrument, en particulier de dplusieurs actionneurs de valve d'un instrument de traitement médical, les actionneurs étant alimentés en pression dans différentes combinaison pendant le fonctionnement normal de l'instrument,
**caractérisé en ce que**
pour plusieurs combinaisons différentes d'actionneurs alimentés en pression, la chute de pression se produisant en commun sur les actionneurs alimentés en pression dans la combinaison respective pendant une phase de fonctionnement stationnaire est mesurée et
dans lequel, pour des actionneurs individuels et/ou des groupes individuels d'actionneurs une valeur d'étanchéité est respectivement déterminée, dans laquelle est intégrée la chute de pression mesurée pour les combinaisons dans lesquelles l'actionneur respectif et/ou le groupe respectif d'actionneurs a été alimenté en pression.

2. Procédé selon la revendication 1, dans lequel, en plus de la chute de pression, la durée de mesure, pendant laquelle la chute de pression s'est produite, est respectivement intégrée dans la valeur d'étanchéité et dans lequel une chute de pression par unité de temps formée à partir des valeurs de chute de pression mesurées et des durées de mesure est de préférence intégrée dans la valeur d'étanchéité.

3. Procédé selon l'une des revendications précédentes, dans lequel, pour déterminer la valeur d'étanchéité d'un seul actionneur et/ou d'un seul groupe d'actionneurs, une valeur moyenne est formée, dans laquelle sont intégrées la chute de pression et de manière avantageuse la durée de mesure pour toutes les combinaisons d'actionneurs alimentés en pression dans lesquelles l'actionneur respectif et/ou le groupe respectif d'actionneurs a été alimenté en pression.

4. Procédé selon la revendication 3, dans lequel, pour déterminer la valeur d'étanchéité d'un seul actionneur et/ou d'un seul groupe d'actionneurs, la valeur moyenne éventuellement pondérée de la chute de pression et/ou de la chute de pression par unité de temps de toutes les combinaisons d'actionneurs alimentés en pression dans lesquelles l'actionneur respectif ou le groupe respectif d'actionneurs a été alimenté en pression est calculée, dans lequel de préférence la durée des phases de fonctionnement stationnaires, pour lesquelles la chute de pression et/ou la chute de pression par unité de temps a été déterminée, est intégrée dans la formation de la valeur moyenne.

5. Procédé selon l'une des revendications précédentes, dans lequel, pour déterminer la valeur d'étanchéité d'un seul actionneur et/ou d'un seul groupe d'actionneurs, la somme des chutes de pression et la somme des durées de mesure pour toutes les combinaisons d'actionneurs alimentés en pression dans lesquelles l'actionneur respectif et/ou le groupe respectif d'actionneurs a été alimenté en pression sont déterminées.

6. Procédé selon la revendication 4, dans lequel le nombre des actionneurs alimentés en pression dans une combinaison définie est intégré dans la formation de la valeur moyenne ou dans lequel la chute de pression commune mesurée pour une combinaison définie d'actionneurs alimentés en pression est intégrée dans la formation de la valeur moyenne indépendamment du nombre des actionneurs alimentés en pression dans cette combinaison.

7. Procédé selon l'une des revendications précédentes, dans lequel la chute de pression commune mesurée pour une combinaison définie d'actionneurs alimentés en pression est intégrée pour tous les actionneurs alimentés en pression de la combinaison respective à hauteur égale dans leur valeur d'étanchéité et dans lequel les valeurs d'étanchéité des actionneurs individuels et/ou des groupes individuels d'actionneurs sont actualisées de manière avantageuse en continu pendant le fonctionnement de l'instrument, dans lequel, pour le contrôle et/ou la surveillance des actionneurs, la gamme des valeurs d'étanchéité déterminées est en outre prise en compte de manière avantageuse ou dans lequel, pour le contrôle et/ou la surveillance des actionneurs, la somme de toutes les valeurs d'étanchéité déterminées est d'autre part prise en compte de manière avantageuse.

8. Procédé selon l'une des revendications précédentes, dans lequel, pour le contrôle et/ou la surveillance des actionneurs, la modification dans le temps des valeurs d'étanchéité déterminées est prise en compte.

9. Procédé selon l'une des revendications précédentes, dans lequel, en cas de détection d'une fuite, le fonctionnement courant est arrêté et un test est effectué, un test initial étant de préférence effectué, selon lequel tous les actionneurs sont contrôlés et/ou dans lequel un test est en outre effectué de manière avantageuse, selon lequel un seul actionneur et/ou un seul groupe d'actionneurs, pour lequel une fuite a été détectée, est contrôlé.

10. Procédé selon l'une des revendications précédentes, dans lequel, en cas de détection et/ou de confirmation d'une fuite, l'actionneur non étanche n'est plus utilisé ou l'instrument passe dans un état de sécurité.

11. Procédé selon l'une des revendications précédentes, dans lequel l'instrument est utilisé pour la commande des valves d'une cassette pour le transport d'un liquide médical, en particulier dans la dialyse.

12. Instrument, en particulier instrument de traitement médical, comprenant plusieurs actionneurs à commande pneumatique ou hydraulique, en particulier plusieurs actionneurs de valve, un appareil de mesure de la pression destiné à la mesure de la pression appliquée en commun sur les actionneurs alimentés en pression, et un dispositif de commande électronique, qui commande les actionneurs et l'appareil de mesure de la pression de telle manière
que les actionneurs sont alimentés en pression dans des combinaisons différentes pendant le fonctionnement normal de l'instrument et pour plusieurs combinaisons différentes d'actionneurs alimentés en pression, la chute de pression se produisant en commun sur les actionneurs alimentés en pression dans la combinaison respective pendant une phase de fonctionnement stationnaire est mesurée,
dans lequel une unité d'évaluation est prévue, qui, pour des actionneurs individuels et/ou des groupes individuels d'actionneurs détermine respectivement une valeur d'étanchéité, dans laquelle est intégrée la chute de pression mesurée pour les combinaisons dans lesquelles l'actionneur respectif et/ou le groupe respectif d'actionneurs a été alimenté en pression, et
dans lequel une unité de contrôle et/ou de surveillance contrôle et/ou surveille l'étanchéité des actionneurs de l'instrument sur la base des valeurs d'étanchéité déterminées.

13. Instrument selon l'une des revendications précédentes, dans lequel l'unité d'évaluation forme, pour déterminer la valeur d'étanchéité d'un seul actionneur et/ou d'un seul groupe d'actionneur, une valeur moyenne, dans laquelle est intégrée la chute de pression et de manière avantageuse la durée de mesure pour toutes les combinaisons d'actionneurs alimentés en pression dans lesquelles l'actionneur respectif et/ou le groupe respectif d'actionneurs a été alimenté en pression, dans lequel l'unité d'évaluation calcule de préférence la valeur d'étanchéité d'un seul actionneur et/ou d'un seul groupe d'actionneurs comme valeur moyenne éventuellement pondérée de la chute de pression et/ou de la chute de pression par unité de temps de toutes les combinaisons d'actionneurs alimentés en pression dans lesquelles l'actionneur respectif ou le groupe respectif d'actionneurs a été alimenté en pression, dans lequel au choix la durée des phases de fonctionnement stationnaires, pour lesquelles la chute de pression et/ou la chute de pression par unité de temps a été déterminée, est de préférence intégrée dans la formation de la valeur moyenne et dans lequel au choix le nombre des actionneurs alimentés en pression dans une combinaison définie est intégré dans la formation de la valeur moyenne ou dans lequel au choix la chute de pression commune mesurée pour une combinaison définie d'actionneurs alimentés en pression est intégrée dans la formation de la valeur moyenne indépendamment du nombre des actionneurs alimentés en pression dans cette combinaison.

14. Instrument selon l'une des revendications précédentes, dans lequel la chute de pression commune mesurée pour une combinaison définie d'actionneurs alimentés en pression est intégrée pour tous les actionneurs alimentés en pression dans la combinaison respective à hauteur égale dans leur valeur d'étanchéité.

15. Instrument selon l'une des revendications précédentes, dans lequel l'unité d'évaluation, pour déterminer la valeur d'étanchéité d'un seul actionneur et/ou d'un seul groupe d'actionneurs, détermine la somme des chutes de pression et la somme des durées de mesure pour toutes les combinaisons d'actionneurs alimentés en pression dans lesquelles l'actionneur respectif et/ou le groupe respectif d'actionneurs a été alimenté en pression.

16. Instrument selon l'une des revendications précédentes, dans lequel, en cas de détection d'une fuite, l'unité de contrôle et/ou de surveillance arrête le fonctionnement courant et effectue un test, un test initial étant de préférence effectué, selon lequel tous les actionneurs sont contrôlés.

17. Instrument selon la revendication 16, dans lequel un test est effectué, selon lequel un seul actionneur et/ou un seul groupe d'actionneurs, pour lequel une fuite a été détectée, est contrôlé.

18. Instrument selon l'une des revendications précédentes, dans lequel le dispositif de commande, en cas de détection et/ou de confirmation d'une fuite, n'utilise plus l'actionneur non étanche ou commute l'instrument dans un état de sécurité.

19. Instrument selon l'une des revendications précédentes, destiné à la commande des valves d'une cassette pour le transport d'un liquide médical, en particulier pour l'utilisation dans la dialyse.

20. Instrument selon l'une des revendications précédentes, comprenant une surface de couplage, à laquelle une cassette pour le transport d'un liquide médical peut être couplée, les actionneurs étant disposés sur la surface de couplage.

21. Produit programme informatique, en particulier support de stockage comprenant un programme informatique, en particulier destiné à être exécuté sur un instrument, comprenant des instructions pour l'exécution d'un procédé selon l'une des revendications précédentes.
